# EUROPÄISCHE PATENTANMELDUNG

(11) **EP 1 889 825 A1**
(43) Veröffentlichungstag der Anmeldung: **20.02.2008**
(21) Anmeldenummer: 06017124.6
(22) Anmeldetag: 17.08.2006
(51) Int. Cl.: C07C 29/132, C07C 31/20

(54) **Verfahren zur Herstellung von 1,2-Propandiol**

(71) Anmelder: Cognis Oleochemicals GmbH, 40589 Düsseldorf (DE)
(72) Erfinder: Westfechtel, Alfred, 40724 Hilden (DE); Grundt, Elke, 40589 Düsseldorf (DE); Alexandre, Teresa, 47802 Krefeld (DE); Klein, Norbert, 40822 Mettmann (DE)
(74) Vertreter: Herzog, Martin

(57) **Zusammenfassung**

Vorgeschlagen wird ein Verfahren zur Herstellung von 1,2-Propandiol mit einer Reinheit von mindestens 98%, wobei man in einem ersten Schritt Glycerin in Gegenwart eines heterogenen Kupfer-haltigen Katalysators, insbesondere eines heterogenen Kupfer-Chrom-haltigen Katalysators, kontinuierlich hydriert, wobei man Glycerin zu höchstens 95% umsetzt, und die im ersten Schritt erhaltene Reaktionsmischung in einem zweiten Schritt einer Destillation unterwirft.

## Beschreibung

### Gebiet der Erfindung

Die Erfindung betrifft ein Verfahren zur Herstellung von 1,2-Propandiol.

### Stand der Technik

Das durch Spaltung von Fetten und Ölen reichlich als industrieller Rohstoff zur Verfügung stehende Glycerin kann als Basis für verschiedene Synthesen dienen. Erwähnt seien in diesem Zusammenhang einerseits die Dehydratisierung (Wasserabspaltung) zu Acetol (vergleiche zum Beispiel DE-A-41,28,692 / Henkel; Fleckenstein et al.) und andererseits die unter Wasserabspaltung verlaufende Hydrierung zu 1,2-Propandiol.

1,2-Propandiol ist ein industriell interessanter Rohstoff. Es wird in einer Vielzahl von Anwendungen eingesetzt, nämlich in der Nahrungsmittelindustrie, als Lösungsmittel für Farbstoffe und Aromen, als Feuchthaltemittel für Tabak, in Kosmetika, als Bestandteil von Brems- und hydraulischen Flüssigkeiten, Frostschutzmitteln, Schmiermitteln in Kältemaschinen, als Lösungsmittel für Fette, Öle, Harze, Wachse, Farbstoffe usw. Es dient auch als Ausgangsprodukt zur Herstellung anderer Produkte. Durch Veresterung, und/oder Veretherung lassen sich zahlreiche, als Lösungsmittel, zu Synthesen, als Weichmacher, Verdickungsmittel, Emulgatoren usw. verwendbare Produkte gewinnen. In der Mehrzahl der genannten Anwendungen spielt die Reinheit des 1,2-Propandiols eine Rolle. 1,2-Propandiol wird üblicherweise durch Hydratisierung von Propylenoxid industriell hergestellt. Die oben erwähnte Herstellung von 1,2-Propandiol durch Hydrierung von Glycerin ist zwar mehrfach beschrieben worden, hat sich jedoch in der industriellen Praxis bislang nicht durchsetzen können.

Das älteste der Anmelderin bekannte Patentdokument, das die Hydrierung von Glycerin zu 1,2-Propandiol beschreibt ist das deutsche Patent DE-C-524,101 von I.G.Farben. Gemäß Beispiel 1 dieser Schrift wird ein Wasserstoffstrom, der 1 bis 1,5 Vol.-% Glycerin enthält, bei 200 bis 210°C über einen Katalysator geleitet, der auf Glasperlen aufgebracht ist. Dabei wird eine Flüssigkeit erhalten, die nach Entfernung des bei der Hydrierung gebildeten Wassers fraktioniert destilliert wird. Die Umsetzung des Glycerins war nahezu vollkommen. Bei der Destillation wurde ein Gemisch von 1,2-Propandiol und Propylalkohol erhalten. Die Ausbeute an 1,2-Propandiol wurde mit 70% angegeben.

DE-C-541,362 offenbart die Hydrierung von Polyoxyverbindungen. Dabei werden flüssige oder feste Polyoxyverbindungen in wässriger Lösung oder Suspension mit Wasserstoff in Gegenwart von Katalysatoren behandelt. In Beispiel 1 werden Glycerin und ein Nickel-Katalysator in einer Bombe bei 200 bis 240 °C und einem Druck von 100 atm mit Wasserstoff behandelt. Mithin handelt es sich um ein Batch-Verfahren, also eine diskontinuierliche Hydrierung, wobei der Katalysator in Pulverform im flüssigen Glycerin aufgeschlämmt ist. Aus der Angabe, dass nach 4 Stunden die errechnete Menge an Wasserstoff aufgenommen wurde, wird für den Fachmann deutlich, dass Glycerin offensichtlich quantitativ umgesetzt wurde.

Lange Zeit lag das Thema der Glycerinhydrierung zu 1,2-Propandiol offenbar brach. Erst etwa 50 Jahre später wurde es wieder aufgegriffen:
Montassier et al. berichten in Heterogeneous Catalysis and Fine Chemicals (1988, Elsevier, Amsterdam, Seiten 165-170), dass die Hydrierung von Glycerin in Gegenwart von Ru-, Ni-, Rh- bzw. Ir-basierten Katalysatoren zu Kohlenwasserstoffen, insbesondere Methan, führt. Mit Raney-Kupfer als Katalysator wird dagegen eine Mischung von 1,2-Propandiol, Ethylenglykol und Kohlendioxid erhalten.
EP-A-523,014 und EP-A-523,015 (Novamont) gehen das Problem der mangelnden Selektitivät der Glycerinhydrierung zu 1,2-Propandiol dadurch an, dass sie einerseits spezielle Katalysatoren auf Ruthenium- bzw. Zink-Kupfer-Basis in sehr verdünntem wässrigem Milieu durchführen. Gleichwohl lässt die Selektivität sehr zu wünschen übrig:
   Gemäß den Beispielen der EP-A-523,015 (Einsatz eines Zink-Kupfer-Kataölysators) werden bei der Hydrierung von Glycerin neben Propylenglykol große Mengen an Ethylenglykol und außerdem erhebliche Mengen an Milchsäure (lactic acid) gebildet.
   Gemäß den Beispielen der EP-A-523,014 (Einsatz eines Sulfid-modifizierten Ruthenium-Katalysators) werden bei der Hydrierung von Glycerin neben Propylenglykol sehr große Mengen an Ethylenglykol gebildet.
   Außerdem ist eine Verdünnung des einzusetzenden Rohstoffes (Glycerin) mit Wasser technisch unbefriedigend, weil dieses Wasser anschließend aus dem Reaktionsgemisch entfernt werden muss.

DE-A-43,02,464 von 1993 (Henkel; Fleckenstein et al.) beschrieb die kontinuierliche Hydrierung von Glycerin in Gegenwart eines speziellen heterogenen Katalysators. Vorzugsweise kam dabei ein kupferhaltiger Katalysator zum Einsatz, insbesondere Kupferchromit. Gemäß den Ausführungsbeispielen wurde Glycerin im wesentlichen quantitativ umgesetzt (Glycerinanteil in der Reaktionsmischung unterhalb von 0,1 %), lediglich 3 der insgesamt 10 Beispiele arbeiteten mit einem Teilumsatz von Glycerin (Beispiele 1, 3 und 9). Für sämtliche Reaktionsmischungen der Beispiele wurden zwar analytische Daten angegeben, die Gemische wurden jedoch nicht aufgearbeitet. Mithin ist die technische Lehre der DE-A-43,02,464 darin zu sehen, dass sie die prinzipielle Eignung von Kupferhaltigen heterogenen Katalysatoren zur Hydrierung von Glycerin zu 1,2-Propandiol aufzeigt. Sie schließt nicht die Lehre ein, dass eine Teilhydrierung von Glycerin gefolgt von einer Destillation zu einem 1,2-Propandiol mit ganz speziellem Eigenschafts-Profil führt. Die Schrift vermerkt auf Seite 3, Zeilen 16/17 lediglich, dass zur Aufarbeitung des bei der Hydrierung erhaltenen Reaktionsgemisches Wasser abgezogen werde; die rein optionale Möglichkeit "weiterer Aufarbeitungsschritte" wird nur am Rande erwähnt ohne dass in irgendeiner Weise darauf eingegangen wird, wie eine solche Aufarbeitung auszusehen hätte und ob es Aufarbeitungsschritte gibt, die von besonderer Relevanz wären.

Gemäß US-A-5,616,817 (BASF; Schuster und Eggersdorfer) von 1995 wird die Glycerinhydrierung so durchgeführt, dass einerseits Glycerin mit einem Wassergehalt von bis zu 20 Gew.-% und andererseits spezielle Katalysatoren (mit einem Gehalt von 40-70% Co, 10-20% Mn, 0-10% Mo und 0-10% Cu) zum Einsatz kommen. Glycerin wird dabei vollständig umgesetzt. Doch auch mit dem hier offenbarten speziellen Katalysator stößt man immer noch an Grenzen der Selektivität. Dies wird dem Fachmann sofort deutlich, wenn er die beiden Ausführungsbeispiele betrachtet. In Beispiel 1 enthält die Reaktionsmischung 95,8% 1,2-Propandiol und 3,2% n-Propanol, in Beispiel 2 enthält die Reaktionsmischung 92% 1,2-Propandiol und 4,3% n-Propanol en

In US-A-6,479,713 (Battelle Memorial Institute) aus dem Jahre 2002 beschreibt die Hydrierung von Zuckern oder Zuckeralkoholen, wobei spezielle Rhenium enthaltende Multimetall-Katalysatoren eingesetzt werden. Als ein möglicher Rohstoff, der zur Hydrierung eingesetzt werden kann, ist in der Beschreibung Glycerin genannt. In den Tabellen 2 bis 4 werden Versuchsergebnisse zusammengestellt, wo Glycerin in Anwesenheit derartiger Katalysatoren hydriert wird. Dabei fällt auf, dass 1,2-Propandiol zwar das Hauptprodukt der Hydrierung ist, jedoch sehr große Mengen an Ethylenglykol und Lactat gebildet werden. Sofern dem eingesetzten Glycerin nicht eine Base zugesetzt wurde, kam im übrigen 1,3-Propandiol als zusätzliches unerwünschtes Nebenprodukt hinzu (Spalte 11, Zeilen 49-51). Die Ergebnisse der Glycerinhydrierung gemäß der US-A-6,479,713 sind als äußerst unbefriedigend einzustufen.

WO-A2-2005/095536 (Suppes et al.) befasst sich mit der Hydrierung von Glycerin zu niederen Alkoholen. Die eigentliche technische Lehre lässt sich der umfangreichen Schrift nicht auf den ersten Blick entnehmen, der Fachmann gewinnt vielmehr den Eindruck, dass hier vieles aus dem Stand der Technik wiederholt wird. Es überrascht daher nicht, wenn der Recherchenbericht WO-A3-2005/095536 acht X-Dokumente und 13 Y-Dokumente auflistet. Worum es den Erfindern geht, scheint prägnanter der "parallelen" Publikation von G.J.Suppes et al. in Applied Catalysis A: General 281 (2005), Seiten 225-231 entnehmbar zu sein: Die Hydrierung von Glycerin bei "milden" Bedingungen um 200 °C und die Herstellung von 1,2-Propandiol in zwei Schritten, nämlich der Herstellung von Acetol aus Glycerin nebst anschließender Hydrierung des Acetols zu 1,2-Propandiol.

### Beschreibung der Erfindung

Aufgabe der vorliegenden Erfindung war es, ein neues Verfahren zur Herstellung von 1,2-Propandiol durch Hydrierung von Glycerin bereitzustellen.
Das so hergestellte 1,2-Propandiol sollte sich durch eine hohe Reinheit, insbesondere eine Reinheit von mindestens 98% und vorzugsweise von mindestens 99% auszeichnen.
Darüber hinaus sollte das so hergestellte 1,2-Propandiol von möglichst hoher geruchlicher Qualität sein, worunter zu verstehen ist, dass es keinesfalls einen unangenehmen und/oder starken Geruch, sondern allenfalls einen leichten Geruch aufweisen und vorzugsweise geruchlos sein sollte.

Die genannte komplexe Aufgabe wurde durch das nachstehend beschriebene erfindungsgemäße Verfahren in ausgezeichneter Weise gelöst.

Gegenstand der Erfindung ist ein Verfahren zur Herstellung von 1,2-Propandiol mit einer Reinheit von mindestens 98%, wobei man
- in einem ersten Schritt Glycerin in Gegenwart eines heterogenen Kupfer-haltigen Katalysators - insbesondere eines heterogenen Kupfer-Chrom-haltigen Katalysators - kontinuierlich hydriert, wobei man Glycerin zu höchstens 95% umsetzt, und die im ersten Schritt erhaltene Reaktionsmischung
- in einem zweiten Schritt einer Destillation unterwirft.

Unter einer Reinheit von mindestens 98% wird verstanden, dass das nach dem erfindungsgemäßen Verfahren nach Durchlaufen beider Schritte (also Hydrierung und Destillation) ein Produkt erhalten wird, dessen Gehalt an 1,2-Propandiol mindestens 98% beträgt. Die Reinheit wird dabei gaschromatographisch bestimmt (Flächenprozent).

In einer bevorzugten Ausführungsform führt man die beiden Schritte des erfindungsgemäßen Verfahrens so durch, dass die Reinheit des Zielproduktes 1,2-Propandiol mindestens 99% beträgt.

Es sei ausdrücklich festgestellt, dass der Gesamtprozess zur Herstellung von 1,2-Propandiol nach dem erfindungsgemäßen Verfahren zwei Schritte umfasst, nämlich
- die Hydrierung des Glycerins unter den angegebenen Rahmenbedingungen und
- die Aufdestillation des im ersten Schritt erhaltenen Hydrierablaufs zur Abtrennung von Wasser sowie der Verminderung der bei der Hydrierung entstandenen Nebenprodukte.

Dass mit dieser Kombination von Schritten ein solcher Erfolg erzielt wird, der die oben genannte komplexe Aufgabe löst, ist nicht trivial und war für den Fachmann nicht vorauszusehen.
In diesem Zusammenhang ist folgendes aufschlussreich: Führt man die Hydrierung von Glycerin in quantitativer Weise durch, so erhält man Hydrierabläufe die geradezu stinken. Destilliert man anschließend derartige (also bei vollständiger Hydrierung von Glycerin erhaltene) Hydrierabläufe, so stellt man fest, dass die auf diese Weise erhaltenen Fraktionen von 1,2-Propandiol immer noch einen unangenehmen Geruch aufweisen.
Die Untersuchungen der Anmelderin deuten darauf hin, dass die Geruchsträger, die beim erfindungsgemäßen Verfahren mit dem wie oben angegebenen Teilumsatz von Glycerin und die Geruchsträger, die bei vollständigem Umsatz von Glycerin erhalten werden, zwar zum Teil identisch, zum Teil jedoch unterschiedlich sind und im übrigen in unterschiedlichem Ausmaß gebildet werden, mit der Folge, dass sie sich durch Destillation in unterschiedlichem Maße entfernen lassen. Und hier ist das erfindungsgemäße Verfahren einer Vorgehensweise, bei der Glycerin quantitativ hydriert und anschließend destilliert wird, weit überlegen. Dieser überraschende Befund macht den Kern der Lehre der vorliegenden Erfindung aus und konnte vom Fachmann in keiner Weise dem Stand der Technik entnommen werden noch war er durch Dokumente des Standes der Technik nahe gelegt.

Im Übrigen hat sich herausgestellt, dass beim erfindungsgemäßen Verfahren mit der genannten Teilhydrierung in Schritt 1 ein geringerer Gehalt des destillativ schlecht zu entfernenden und unerwünschten Nebenprodukts Ethylenglykol erzielt wird.

Vorzugsweise setzt man in Schritt 1 des erfindungsgemäßen Verfahrens (Hydrierung) Glycerin zu höchstens 90% um.

Vorzugsweise führt man Schritt 1 des erfindungsgemäßen Verfahrens (Hydrierung) in Abwesenheit eines organischen Lösungsmittels durch.

Das erfindungsgemäße Verfahren wird in Gegenwart eines heterogenen Kupferhaltigen Katalysators durchgeführt. Besonders bevorzugt ist es, einen heterogenen Kupfer-Chrom-haltigen Katalysator einzusetzen. Dabei ist es besonders bevorzugt, einen Kupferchromit-Katalysator einzusetzen; dieser enthält 30 bis 40 Gew.-% Kupfer, 23 bis 30 Gew.-% Chrom, bezogen jeweils auf die oxidische Katalysatormasse, sowie gewünschtenfalls weitere Übergangsmetalle in Form ihrer Oxide. In einer Ausführungsform kann ein solcher Kupferchromit-Katalysator 1 bis 7 Gew.-%, insbesondere 1,5 bis 3 Gew.-% Barium, bezogen auf die oxidische Katalysatormasse enthält. In einer Ausführungsform enthält der Katalysator 32 bis 38 Gew.-% Kupfer, bezogen auf die oxidische Katalysatormasse, 26 bis 30 Gew.-% Chrom, 1,5 bis 3 Gew.-% Barium, 0,5 bis 2 Gew.-% Silicium und zusätzlich je 1 bis 5, bevorzugt 2 bis 3 Gew.-%, Mangan, Zirkon und/ oder Cer, bezogen auf die oxidische Katalysatormasse. Dieser Katalysator und sein Herstellungsverfahren ist ausführlich in der EP-A-254,189 beschrieben. Auf die dort enthaltene Offenbarung wird hier ausdrücklich Bezug genommen, und die dort gegebenen Informationen sollen auch Bestandteil der vorliegenden Anmeldung sein.

Es ist bevorzugt, dass der Katalysator eine hohe Oberfläche und Porösität aufweist, so dass eine hohe Aktivität und Selektivität sowie eine für technische Anwendungen besonders wichtige hohe Standzeit erreicht wird. So ist es vorteilhaft, wenn der verwendete Katalysator eine spezifische Oberfläche im Bereich von 20 bis 80 m²/g, bevorzugt 25 bis 70 m²/g sowie ein Porenvolumen im Bereich von 0,1 bis 1,0 cm³/g aufweist.

Schritt 1 des erfindungsgemäßen Verfahrens wird vorzugsweise so durchgeführt: Man setzt zur Hydrierung verdünnten oder unverdünnten Wasserstoff ein, wobei man bei Drucken im Bereich von 20 bis 300 bar, insbesondere bei 100 bis 250 bar, und bei Temperaturen im Bereich von 150°C bis 280°C, insbesondere 180 bis 220 °C, arbeit; bei der kontinuierlichen Fahrweise, leitet man Glycerin in dampfförmiger oder flüssiger Form über ein Katalysator-Festbett. Vorzugsweise stellt man ein molares Verhältnis von Wasserstoff zu Glycerin im Bereich von 2 bis 500 ein, wobei man überschüssigen Wasserstoff gewünschtenfalls im Kreis fährt.

Schritt 1 des erfindungsgemäßen Verfahrens (Hydrierung) kann in ähnlichen Reaktoren durchgeführt werden, die für die Herstellung von Fettalkoholen durch Hydrierung von Fettsäuremethylester oder direkt aus Triglyceriden üblich und bekannt sind. Vorzugsweise wird Schritt 1 des erfindungsgemäßen Verfahrens (Hydrierung) in isotherm betriebenen Rohrreaktoren oder Rohrbündelreaktoren durchgeführt. Die Reaktionsparameter Temperatur und Druck können dabei der jeweiligen Katalysatoraktivität entsprechend angepasst werden. Die Reaktionswärme wird größtenteils über die Reaktorwand abgeführt, so dass eine praktisch isotherme Fahrweise möglich ist. Dabei ist es wiederum bevorzugt, dass man die Hydrierung so durchführt, dass man flüssiges Glycerin in Rieselfahrweise ("trickle bed") im Gleich- oder Gegenstrom mit Wasserstoff über ein Katalysator-Festbett leitet. In einer weiteren Ausführungsform führt man Glycerin ohne Rückvermischung mit einer definierten Verweilzeit durch die Katalysatorschüttung in dem bzw. den Rektionsrohren.

Das zur Hydrierung eingesetzte Glycerin kann wasserhaltig sein. Vorzugsweise sollte der Wassergehalt jedoch unterhalb von 15 Gew.-% liegen. Dies hat verfahrensökonomische Gründe, denn im Zuge der Hydrierung wird ohnehin Wasser gebildet, das im zweiten Verfahrensschritt im Zuge der Destillation entfernt wird. In einer weiteren Ausführungsform beträgt der Wassergehalt des eingesetzten Glycerins weniger als 2% Gew.-%. Es kann auch gewünscht sein, wasserfreies Glycerin einzusetzen bzw. ein Glycerin, dessen Wassergehalt nur im Spurenbereich liegt.

Gewünschtenfalls kann das beim erfindungsgemäßen Verfahren erhaltene 1,2-Propandiol weiteren, dem Fachmann bekannten Reinigungsschritten unterworfen werden, um die Reinheit des Produktes weiter zu erhöhen.

Gewünschtenfalls kann ein Teil des Hydrierablaufes (also der Reaktionsmischung, die nach Durchlaufen von Schritt 1 erhalten wird) als Verdünnungsmittel für die kontinuierliche Hydrierung (also Schritt 1) benutzt werden. Beispielsweise kann man 25% des Hydrierablaufs dem in Schritt 1 des erfindungsgemäßen Verfahrens eingesetzten Rohstoff (Glycerin) zusetzen, um auf diese Weise eine schonende Reaktionsführung ohne Temperaturspitzen am Hydrierkatalysator zu erzielen. Vorzugsweise führt man jedoch Glycerin ohne Rückvermischung mit einer definierten Verweilzeit durch die Katalysatorschüttung in dem bzw. den Reaktionsrohr(en).

Die beim erfindungsgemäßen Verfahren erhaltenen Hydrierabläufe und ebenso das nach Durchlaufen beider Schritte des erfindungsgemäßen Verfahrens erhaltene 1,2-Propandiol können als Kältemittel verwendet werden.

Ein weiterer Erfindungsgegenstand ist dementsprechend die Verwendung der beim erfindungsgemäßen Verfahren erhaltenen Hydrierabläufe und/oder des nach Durchlaufen beider Schritte des erfindungsgemäßen Verfahrens erhaltenen 1,2-Propandiols als Kältemittel.

### Beispiele

### Beispiel 1:

Ein 2 m langes Reaktionsrohr mit einem Innendurchmesser von 25 mm (kontinuierliche Hydrieranlage) wurde mit einem Katalysator vom Kupfer-Chrom-Typ (Typ G99 BO 3 x 3 der Fa. Südchemie) befüllt. Man hydrierte reines Glycerin kontinuierlich unter folgenden Bedingungen: Temperatur = 220 °C; 13 Nm³/h Wasserstoff; Durchsatz = 882 g/h.
Zum Monitoring des Umsetzungsgrades des Glycerin wurde der Hydrierablaufs per GC analysiert, jedoch nicht vollständig, sondern lediglich bezüglich seines Gehaltes an Glycerin, 1,2-Propandiol und Ethylenglykol. Ergebnis: 34,2% Glycerin, 65,3% 1,2-Propandiol, 0,5% Ethylenglykol. Nebenprodukte konnten nur als kleine Verunreinigungen im GC beobachtet werden. Der Hydrierablauf wies einen nur schwachen Geruch auf und war fast klar.
Der Hydrierablauf wurde im Labor destilliert: Fraktionierung mit aufgesetzter 20 cm Vigreux- Kolonne, Destillation bei Atmosphärendruck, Einsatzmenge: 1000g
Bei der Fraktionierung wurden die vier Fraktionen I bis IV erhalten. Die zugehörigen Daten sind Tabelle 1 zu entnehmen. In dieser Tabelle bedeutet in Spalte 1 "Fr" die jeweilige Fraktion und "R" Destillationsrückstand; in den Spalten 2 und 3 sind Sumpf- und Brüdentemperatur der jeweiligen Fraktionen zusammengestellt; Spalte 4 nennt die Menge (in Gramm) der erhaltenen Fraktionen; Spalte 5 kennzeichnet die Fraktionen näher.

**Tabelle 1**

| **Fr.** | **Sumpf** | **Brüden** | **Menge** | **Bemerkungen** |
|---|---|---|---|---|
| | °C | °C | g | |
| I | 117-178 | 77-106 | 114,8 | Wasser, trüb |
| II | 178-187 | 106-170 | 5,5 | Wasser, klar, riecht nach Ether |
| III | 188-189 | 170-176 | 4,2 | farblos, klar, geruchlos |
| **IV** | **189-215** | **177-187** | **505,4** | **farblos, klar, geruchlos** **(99,5% 1,2-Propandiol, 0,5% Ethylenglykol)** |
| R | | | 367,3 | gelb, schwach trüb 21,0% 1,2-Propandiol, 0,7% Ethylenglykol u. 79,0% Glycerin |

Im Sinne des erfindungsgemäßen Verfahrens ist Fraktion IV (505,4g farblose, klare Flüssigkeit). Bei dieser Fraktion handelt es sich um das Zielprodukt.

### Vergleichsbeispiel 1:

Beispiel 1 wurde wiederholt, jedoch wurde Glycerin praktisch vollständig (und nicht partiell wie in Beispiel 1) hydriert. Dies geschah durch Erhöhung der Temperatur auf 230 °C und durch Reduzierung des Durchsatzes auf 441 g/h.
Zum Monitoring des Umsetzungsgrades des Glycerin wurde der Hydrierablaufs analysiert, jedoch nicht vollständig, sondern lediglich bezüglich seines Gehaltes an Glycerin, 1,2-Propandiol und Ethylenglykol. Ergebnis: 0,5% Glycerin, 98,7% 1,2-Propandiol, 0,8% Ethylenglykol. Der Hydrierablauf wies einen äußerst starken Geruch auf und war trüb. Im GC konnten mehrere kleine Peaks festgestellt werden, die aber nicht qualifiziert werden konnten.

Der Hydrierablauf wurde im Labor destilliert: Fraktionierung mit aufgesetzter 20 cm Vigreux- Kolonne, Destillation bei Atmosphärendruck, Einsatzmenge: 1000g
Bei der Fraktionierung wurden die vier Fraktionen I bis IV erhalten. Die zugehörigen Daten sind Tabelle 2 zu entnehmen.

**Tabelle 2**

| **Fr.** | **Sumpf** | **Brüden** | **Menge** | **Bemerkungen** |
|---|---|---|---|---|
| | °C | °C | g | |
| I | 113-175 | 77-105 | 214,8 | Wasser, trüb |
| II | 178-187 | 106-170 | 26,5 | trüb, riecht nach Ether |
| III | 188-189 | 170-176 | 4,2 | farblos, riecht intensiv |
| **IV** | **189-215** | **177-189** | **705,4** | **farblos, klar, riecht nach Ether,** **(95,5% 1,2-Propandiol, 1,2% Ethylenglykol, 3,3% unbekannte Nebenprodukte)** |
| R | | | 47,3 | braun, trüb 22,0% 1,2-Propandiol, 0,7% Ethylenglykol u. 77,0% Glycerin |

Endprodukt des Vergleichsversuchs ist Fraktion IV (705,4g farblose Flüssigkeit, Geruch nach Ether).

### Beispiel 2:

Beispiel 1 wurde wiederholt, jedoch wurde das eingesetzte Glycerin im Verhältnis 4 : 1 mit dem in Beispiel 1 erhaltenen Hydrierablaufs vermischt (4 Gewichtsteile reines Glycerin und 1 Gewichtsteil des Hydrierablaufs aus Beispiel 1).
Zum Monitoring des Umsetzungsgrades des Glycerin wurde der Hydrierablaufs analysiert, jedoch nicht vollständig, sondern lediglich bezüglich seines Gehaltes an Glycerin, 1,2-Propandiol und Ethylenglykol. Ergebnis: 13,8% Glycerin, 85,6% 1,2-Propandiol, 0,6% Ethylenglykol. Der Hydrierablauf wies einen nur schwachen Geruch auf und war fast klar.

Der Hydrierablauf wurde im Labor destilliert: Fraktionierung mit aufgesetzter 20 cm Vigreux- Kolonne, Destillation bei Atmosphärendruck, Einsatzmenge: 1000g
Bei der Fraktionierung wurden die vier Fraktionen I bis IV erhalten. Die zugehörigen Daten sind Tabelle 3 zu entnehmen.

**Tabelle 3**

| **Fr.** | **Sumpf** | **Brüden** | **Menge** | **Bemerkungen** |
|---|---|---|---|---|
| | °C | °C | g | |
| I | 117-178 | 77-106 | 167,7 | Wasser, trüb, Ether-Geruch |
| II | 178-187 | 106-167 | 10,5 | Wasser, klar, riecht nach Ether |
| III | 188-189 | 168-176 | 9,2 | farblos, klar, leichter Geruch |
| **IV** | **189-215** | **177-187** | **630,7** | **farblos, klar, geruchlos** **(99,4% 1,2-Propandiol, 0,6% Ethylenglykol)** |
| R | | | 181,3 | gelb, schwach trüb 34,0% 1,2-Propandiol, 0,7% Ethylenglykol u. 64,0% Glycerin |

Im Sinne des erfindungsgemäßen Verfahrens ist Fraktion IV (630,7g farblose, klare Flüssigkeit, geruchlos) die entscheidende Fraktion. Es handelt sich hier um das Zielprodukt Produkt. In dieser Fraktion konnten gaschromatographisch keine weiteren Nebenprodukte identifiziert werden.

### Vergleichsbeispiel 2:

Beispiel 1 wurde wiederholt, jedoch wurde Glycerin vollständig (und nicht partiell wie in Beispiel 1) hydriert. Dies geschah einerseits durch Erhöhung der Temperatur auf 250 °C, Einsatz eines Kupfer-Silikat (Cu-SiO₂ / Kata Leuna) und Wahl eines Durchsatzes von 441 g/h.
Der Hydrierablauf (gelb, aufschwimmende Tröpfchen, sehr unangenehmer Geruch) wurde destilliert: Fraktionierung mit aufgesetzter Kolonne wie in Tabelle 1, Destillation bei Atmosphärendruck, Einsatzmenge: 1000g.
Bei der Fraktionierung wurden die fünf Fraktionen I bis V erhalten. Die zugehörigen Daten sind Tabelle 4 zu entnehmen.

**Tabelle 4**

| **Fr.** | **Sumpf** | **Brüden** | **Menge** | **Bemerkungen** |
|---|---|---|---|---|
| | °C | °C | g | |
| **I** | 113-175 | 80-105 | 211,4 | Wasser, enthält 8g org. Phase |
| **II** | 175-191 | 104-181 | 28,5 | leicht trüb, sehr unangenehmer Geruch |
| **III** | 191-192 | 181-187 | 305,8 | fast farblos, klar, leichter Geruch |
| **IV** | 192-205 | 182-188 | 405,4 | farblos, klar, leichter Geruch |
| **V** | 205-230 | 188-189 | 19,4 | farblos, klar, leichter Geruch |
| **R** | | | 29,2 | dunkelbraun, trüb, 27,7% 1,2-Propandiol, 6% Ethylenglykol, 12,1% Glycerin, sehr viele Nebenprodukte |

Die Fraktionen III bis V wurden vereint und ergaben 730,6 g einer klaren Flüssigkeit mit leichtem Geruch. Zusammensetzung laut GC: 93,5% 1,2-Propandiol, 4,0% Ethylenglykol, 2,5% unbekannte Nebenprodukte.
Diese Gesamtfraktion wies faktisch die gleiche Brüdentemperatur auf wie die in den Tabellen 1 bis 3 beschriebene Fraktion IV, d. h. das Destillationsverhalten war identisch.
Als Vergleichsprodukt ist hier die Gesamtfraktion (Fraktionen III bis V) anzusprechen. Es ist deutlich, dass die Qualität der Gesamtfraktion als sehr schlecht einzustufen ist (Reinheit lediglich 93,5%, mit 4% sehr viel Ethylenglykol und ferner 2,5% an weiteren Nebenprodukten). Die Gesamtfraktion hatte außerdem einen Eigengeruch und zeigte bei der Zugabe von Wasser eine leichte Trübung.

## Patentansprüche

1. Verfahren zur Herstellung von 1,2-Propandiol mit einer Reinheit von mindestens 98%, **dadurch gekennzeichnet, dass** man in einem ersten Schritt Glycerin in Gegenwart eines heterogenen Kupfer-haltigen Katalysators kontinuierlich hydriert, wobei man Glycerin zu höchstens 95% umsetzt, und die im ersten Schritt erhaltene Reaktionsmischung in einem zweiten Schritt einer Destillation unterwirft.

2. Verfahren nach Anspruch 1, wobei man einen heterogenen Kupfer-Chrom-haltigen Katalysator einsetzt.

3. Verfahren nach Anspruch 1, wobei man einen heterogenen Kupferchromit-Katalysator einsetzt.

4. Verfahren nach Anspruch 1 bis 3, wobei man die Hydrierung in isotherm betriebenen Rohrreaktoren oder Rohrbündelreaktoren durchführt.

5. Verfahren nach Anspruch 4, wobei man die Hydrierung so durchführt, dass man flüssiges Glycerin in Rieselfahrweise ("trickle bed") im Gleich- oder Gegenstrom mit Wasserstoff über ein Katalysator-Festbett leitet.

6. Verfahren nach Anspruch 5, wobei man Glycerin ohne Rückvermischung mit einer definierten Verweilzeit durch die Katalysatorschüttung in dem bzw. den Rektionsrohren führt.

7. Verfahren nach einem der Ansprüche 1 bis 6, wobei man die Hydrierung bei einer Temperatur im Bereich von 180 bis 220 °C durchführt.

8. Verfahren nach einem der Ansprüche 1 bis 7, wobei der Wassergehalt des eingesetzten Glycerins unterhalb von 2% liegt.

9. Verfahren nach einem der Ansprüche 1 bis 78 wobei man Glycerin im ersten Schritt zu höchstens 90% umsetzt.

10. Verwendung der in Schritt 1 des Verfahrens nach einem der Ansprüche 1 bis 9 erhaltenen Hydrierabläufe und/oder des nach Durchlaufen beider Schritte des Verfahrens nach einem der Ansprüche 1 bis 8 erhaltenen 1,2-Propandiols als Kältemittel.
